# EUROPEAN PATENT APPLICATION

(11) **EP 3 998 054 A1**
(43) Date of publication of application: **18.05.2022**
(21) Application number: 20840995.3
(22) Date of filing: 25.06.2020
(51) Int. Cl.: A61H 1/02, A61F 5/01, A61F 5/02, A61H 3/00, B25J 11/00

(54) **WAIST ASSISTANCE DEVICE**

(30) Priority: 12.07.2019 JP 2019130197
(71) Applicant: Toray Industries, Inc., Tokyo 103-8666 (JP)
(72) Inventor: TAKADA, Nana, Otsu-shi, Shiga 520-2141 (JP); KOYAMA, Yoshiro, Kyoto-shi, Kyoto 604-8175 (JP); SATO, Hirohito, Komaki-shi, Aichi 485-0081 (JP)
(74) Representative: Mewburn Ellis LLP
(86) International application number: PCT/JP2020/025075
(87) International publication number: WO 2021/010129

(57) **Abstract**

An assisting device for waist part 1 has a waist part mounting portion 2 to be mounted to the waist part W of a user; a lower limb mounting portion 3 to be mounted to a part of a lower limb L1, L2 of the user; a hard frame 4 extending between the waist part mounting portion 2 and the lower limb mounting portion 3; and an elastic body 5 extending from the waist part mounting portion 2 toward the lower limb mounting portion 3 on a back side of the user.

## Description

### TECHNICAL FIELD

The invention relates to an assisting device for waist part.

### BACKGROUND ART

In various tasks such as a load carrying task, an agricultural task, a nursing care task and the like, devices to alleviate the burden on the waist part are being used. As one of such devices, Patent document 1 discloses a waist part burden alleviating tool to be worn by being put on the back like a backpack.

The waist part burden alleviating tool of Patent document 1 comprises a frame to be worn on the upper back of a caregiver by being put on the upper back of the caregiver, an attaching/detaching tool for lower limb to be mounted to the lower limb of the caregiver, and an elastic body. The attaching/detaching tool for lower limb is wounded around under the knee joint by tightening using a MAGICTAPE (registered trademark), and the lower end of the frame and the attaching/detaching tool for lower limb are connected by the elastic body. The above-mentioned waist part burden alleviating tool of Patent document 1 allows the burden on the waist part of the caregiver to be alleviated by the restoring force of the elastic body when the caregiver bends the waist part by bending forward and the like.

### PRIOR ART DOCUMENT

### Patent Document

Patent Document 1: JP 2005-192764 A

### SUMMARY OF THE INVENTION

However, in case of the use with a part of the lower limb being tightened as with the waist part burden alleviating tool of Patent document 1, there are some problems. For example, in case that a user such as a caregiver and the like bends forward with the attaching/detaching tool for lower limb being tightened to the lower limb, a force is applied to the attaching/detaching tool for lower limb such that the attaching/detaching tool for lower limb moves upward from the mounting position at which the attaching/detaching tool for lower limb is originally mounted by the restoring force of the elongated elastic body. Therefore, the attaching/detaching tool for lower limb slides up upward from the original mounting position while the nursing care task is repeated.

The above-described sliding up of the attaching/detaching tool for lower limb is not only uncomfortable to the user, but also shorten the separation distance between the lower end of the frame and the attaching/detaching tool for lower limb. In this case, when the user bends forward, the initially-expected amount of elongation of the elastic body is not obtained so that the assisting force due to the restoring force of the elastic body is reduced. Moreover, in case that the attaching/detaching tool for lower limb is firmly tightened to the lower limb in order to prevent sliding up of the attaching/detaching tool for lower limb, sliding up of the attaching/detaching tool for lower limb is somewhat suppressed, but it causes a feeling of pressure or pain due to the firm tightening. Furthermore, in case that the attaching/detaching tool for lower limb is attached at a position at which the attaching/detaching tool for lower limb is difficult to slide up (for example, under the knee, and the like), pain can occur at a part (under the knee, and the like) of the human body, which is caught by the attaching/detaching tool for lower limb. In this way, with the configuration such as the above-described waist part burden alleviating tool, there are problems that the feeling of wearing is uncomfortable at the time of the task and that the assisting force is reduced at the time of the task.

Then, in view of such problems as described above, an object of the invention is to provide an assisting device for waist part which makes it possible to continuously obtain a desired assisting force with a good feeling of wearing.

The assisting device for waist part according to the invention, which solves the above-described problems, mainly comprises the following configurations.

An assisting device for waist part comprising a waist part mounting portion to be mounted to the waist part of a user; a lower limb mounting portion to be mounted to a part of a lower limb of the user, a hard frame extending between the waist part mounting portion and the lower limb mounting portion, and an elastic body extending from the waist part mounting portion toward the lower limb mounting portion on a back side of the user.

### BRIEF DESCRIPTION OF THE DRAWINGS

FIG. 1 is a front view showing a mounting state of an assisting device for waist part according to one embodiment of the invention.
FIG. 2 is a rear view showing the mounting state of the assisting device for waist part according to one embodiment of the invention.
FIG. 3 is a side view showing the mounting state of the assisting device for waist part according to one embodiment of the invention.
FIG. 4 is a front view of the assisting device for waist part of FIG. 1, in a state where a waist part belt and a tightening belt are removed.
FIG. 5 is a side view of the assisting device for waist part of FIG. 1, in a state where the waist part belt and the tightening belt are removed.
FIG. 6 is a perspective view of the assisting device for waist part of FIG. 1, in a state where the waist part belt and the tightening belt are removed.
FIG. 7 is a view showing a false joint portion provided between a waist part mounting portion and a hard frame of the assisting device for waist part of FIG. 1.
FIG. 8 is a side view showing the assisting device for waist part when a user is brought to be in a forward bending posture from the state shown in FIG. 3.

### EMBODIMENT FOR CARRYING OUT THE INVENTION

Below, with reference to the drawings, an assisting device for waist part according to one embodiment of the invention will be described. The embodiment shown below is merely one example, and the assisting device for waist part according to the invention is not limited to the embodiment below.

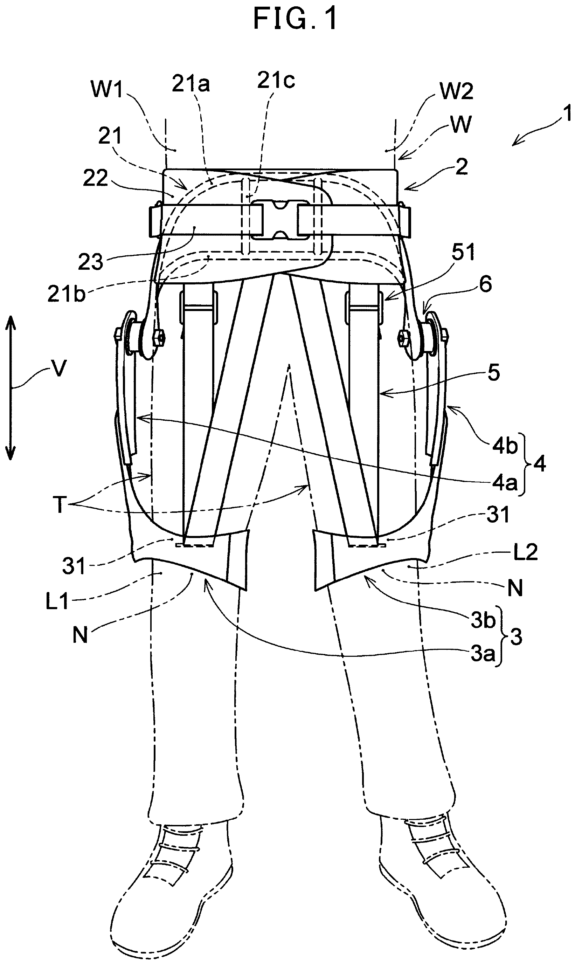

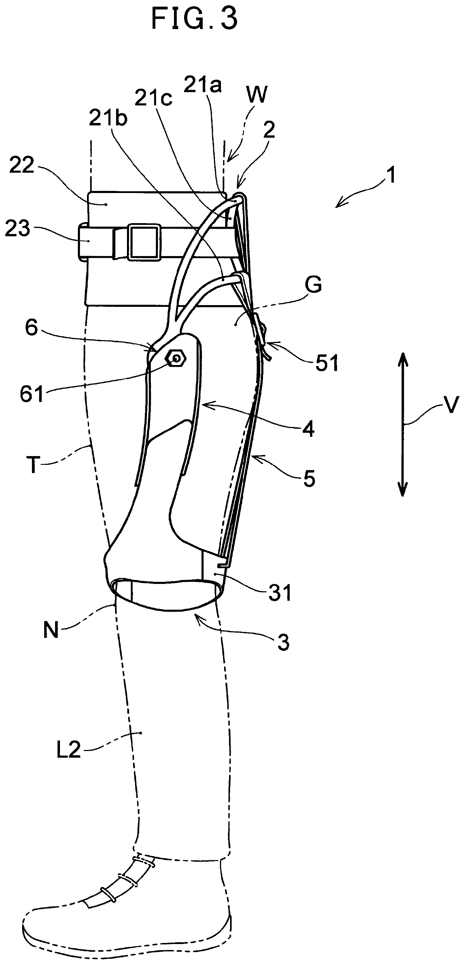

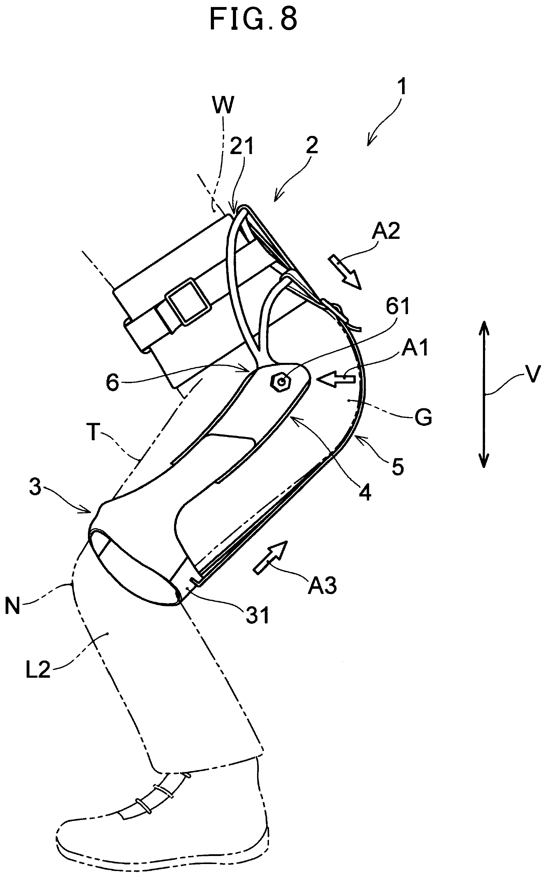

As shown in FIGS. 1 to 3, an assisting device for waist part 1 according to the embodiment comprises a waist part mounting portion 2, a lower limb mounting portion 3, a hard frame 4, and an elastic body 5. As described below, when a user bends forward from an upright posture, returns from a forward bending posture to the upright posture (see FIGS. 3 and 8), and the like, the assisting device for waist part 1 assists muscle tension of the human body by the elastic force of the elastic body 5 being elongated, and alleviates the burden applied to a waist part W (and a thigh T).

The use of the assisting device for waist part 1 is not particularly limited. The assisting device for waist part 1 is used for various tasks such as a load carrying task, an agricultural task, a nursing care task, domestic tasks, and the like, for example to alleviate the burden on the waist part W. The assisting device for waist part 1 is configured to be wearable over a clothing with the user wearing the clothing. The assisting device for waist part 1 according to the embodiment does not use the air pressure, the power of a motor, and the like for assisting of the waist part W, and assisting by the elastic force of the elastic body 5 is basically intended in the assisting device for waist part 1.

Below, each configuration of the assisting device for waist part 1 according to the embodiment will be described.

As shown in FIGS. 1 to 3, the waist part mounting portion 2 is a portion to be mounted to the waist part W of the user in the assisting device for waist part 1. In FIGS. 1 to 3, the user is shown with chain double-dashed lines.

As long as the waist part mounting portion 2 may be mounted to the waist part W of the user, the shape and structure of the waist part mounting portion 2 are not particularly limited. In the embodiment, the waist part mounting portion 2 is wound around and mounted to the waist part W. Specifically, the waist part mounting portion 2 comprises a base portion 21 to be mounted on the back side of the waist part W and a waist part belt 22 to be wound around the waist part W.

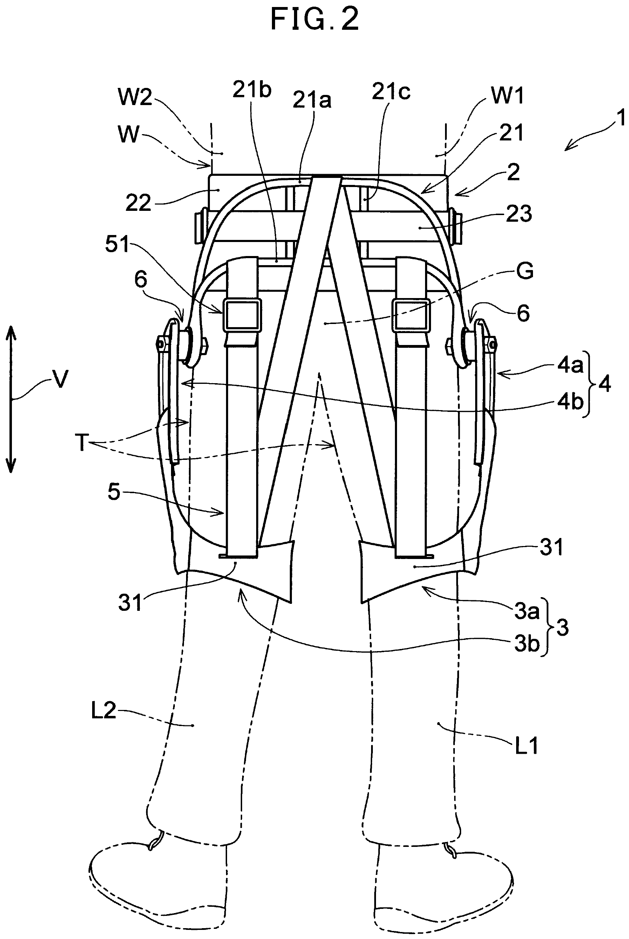

The base portion 21 is mounted on the back side of the waist part W. In the embodiment, the base portion 21 is configured to be mounted on the back side of the waist part W at upper side of a buttocks G of the user without covering the buttocks G of the user (see FIGS. 2 and 3). Although a material forming the base portion 21 is not particularly limited, the base portion 21 may be formed of a hard material having the rigidity such that the base portion 21 is not broken when tension is applied to the base portion 21 from the elastic body 5 connected to the base portion 21, for example. Therefore, when the elastic body 5 is connected to the base portion 21 and the tension is applied to the elastic body 5, it is easy to keep the position of the elastic body 5 on the waist part W side at a desired position. The material forming the base portion 21 may be, for example, metal such as aluminum, iron, titanium, and the like, or hard resin such as nylon, vinyl chloride, acryl, ABS, polycarbonate, polypropylene, polyethylene, carbon, fiber-reinforced plastic (FRP), and the like.

As shown in FIGS. 2 and 3, in the embodiment, the base portion 21 has an upper frame 21a and a lower frame 21b. The upper frame 21a and the lower frame 21b are curved toward the lower limb L1, L2 at positions corresponding to both side portions W1, W2 of the waist part W and are converged. The base portion 21 comprises (a plurality of) reinforcing frames 21c to connect the upper frame 21a and the lower frame 21b in a vertical direction V.

The waist part belt 22 is wound around the waist part W and holds the waist part mounting portion 2 at a predetermined position of the waist part W. In the embodiment, the waist part belt 22 is attached to the base portion 21. When the waist part belt 22 is wound around the waist part W, the base portion 21 is held at the predetermined position. The waist part belt 22 is configured as a band-shaped belt configured to overlap at a front side of the waist part W as shown in FIG. 1, for example. The waist part belt 22 is configured to be stretchable with elastic yarn being inserted into cloth formed of synthetic fiber and the like, for example. The waist part belt 22 has a hook-and-loop fastener (not shown) at ends thereof, so that the waist part belt 22 may be wound around the waist part W with a desired tension. As shown in FIGS. 1 to 3, the waist part mounting portion 2 may have a tightening belt 23 in addition to the waist part belt 22. The tightening belt 23 has buckles at ends of the tightening belt 23 and is configured to adjust the length of the tightening belt 23, making it possible to fix the waist part mounting portion 2 to the waist part W in a desired tightening state. This tightening belt 23 makes it possible to mount the waist part mounting portion 2 further stably to the waist part W.

As long as the waist part mounting portion 2 may be mounted to the waist part W of the user, the waist part mounting portion 2 may be configured to have only one of the base portion 21 and the waist part belt 22.

The lower limb mounting portion 3 is a portion to be mounted to a part of the lower limb L1, L2 of the user in the assisting device for waist part 1. In the embodiment, as shown in FIGS. 1 and 2, the lower limb mounting portion 3 comprises a first lower limb mounting portion 3a to be mounted to a first lower limb (right leg) L1 and a second lower limb mounting portion 3b to be mounted to a second lower limb (left leg) L2.

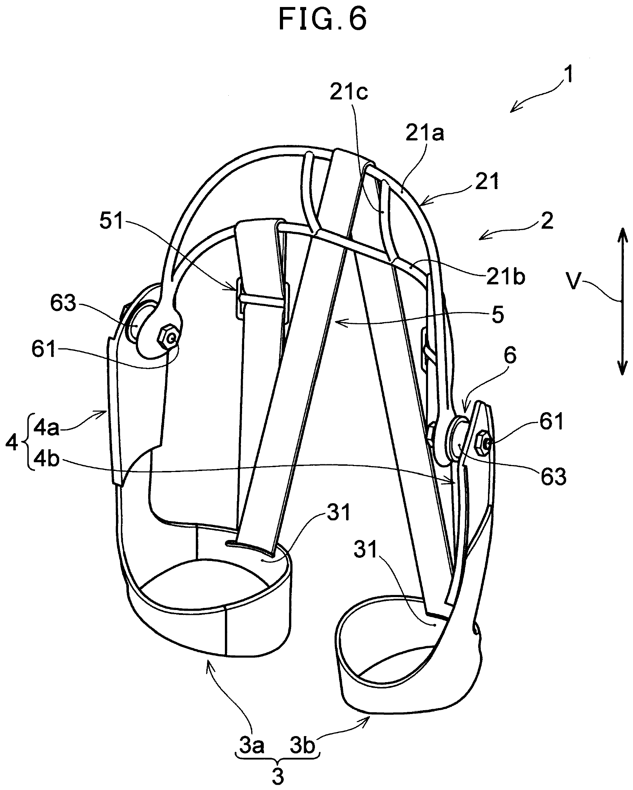

The lower limb mounting portion 3 is mounted so as to cover a part of the outer periphery of the lower limb L1, L2 such that the lower limb mounting portion 3 follows the movement of the lower limb L1, L2 when the lower limb L1, L2 of the user is moved. In the embodiment, the lower limb mounting portion 3 is formed annularly (see FIG. 6) and surrounds the entire outer periphery of the lower limb L1, L2. As long as the lower limb mounting portion 3 can follow the movement of the lower limb L1, L2, the lower limb mounting portion 3 may have a gap in a part of the outer periphery, for example, in case that a cross section perpendicular to the vertical direction V of the lower limb mounting portion 3 is substantially C-shaped. Although illustration is omitted, the lower limb mounting portion 3 is configured such that a part of the lower limb mounting portion 3 is capable of being opened/closed so that the lower limb mounting portion 3 is easily mounted to the lower limb L1, L2. For example, a part of the lower limb mounting portion 3 may be opened/closed in case that the lower limb mounting portion 3 has a hinge portion to allow an opening/closing portion (front portion) of the lower limb mounting portion 3 to be opened/closed with respect to a base (back portion), and a locking portion (for example, a buckle) to fix the opening/closing portion of the lower limb mounting portion 3 to the base in a closed state. Please note that the configuration to allow the lower limb mounting portion 3 to be opened/closed is not particularly limited.

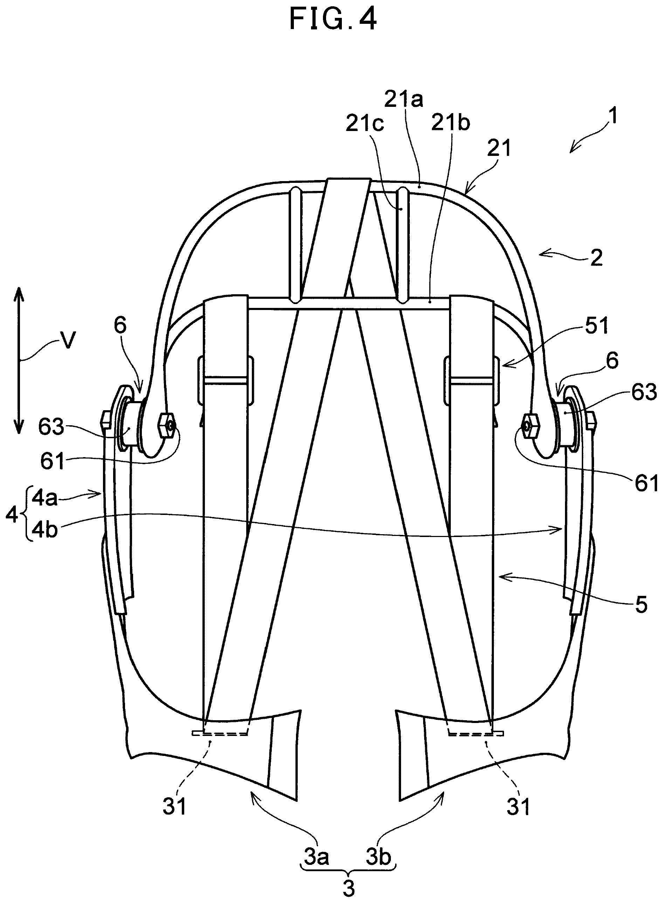

As shown in FIGS. 4 to 6, the lower limb mounting portion 3 is connected to the hard frame 4. In the embodiment, the lower limb mounting portion 3 is connected to the hard frame 4 in a state in which a movement of the lower limb mounting portion 3 in the vertical direction V is regulated. In the embodiment, the lower limb mounting portion 3 is integrally molded with respect to the hard frame 4. However, as long as the lower limb mounting portion 3 is connected to the hard frame 4 such that the movement in the vertical direction V of the lower limb mounting portion 3 with respect to the hard frame 4 is regulated, it is not necessary to be integrally molded with respect thereto.

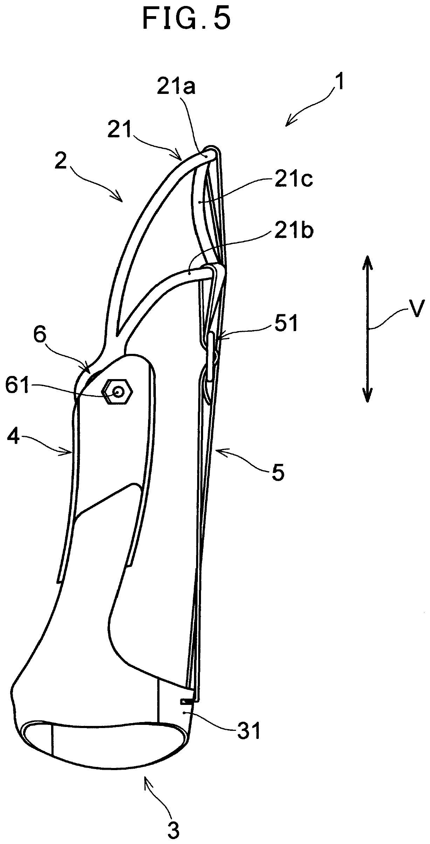

Moreover, as shown in FIGS. 4 to 6, the lower limb mounting portion 3 is connected to the waist part mounting portion 2 via the elastic body 5. The lower limb mounting portion 3 has an elastic body attaching portion 31 to which the elastic body 5 is attached (see FIGS. 5 and 6). The elastic body attaching portion 31 is provided at a rear portion, which is the back side, of the mounting portion 3. In the embodiment, the elastic body attaching portion 31 is a part having a slit provided at the rear portion of the lower limb mounting portion 3 and is configured such that the elastic body 5 can be inserted therethrough. As long as the elastic body attaching portion 31 can maintain the attaching state of the elastic body 5 with tension being applied to the elastic body 5, the shape and the structure of the elastic body attaching portion 31 is not particularly limited.

In the embodiment, the elastic body attaching portion 31 is formed with a hard material and is connected to the hard frame 4 in a state in which a relative movement of the elastic body attaching portion 31 in the vertical direction V with respect to the hard frame 4 is regulated. Therefore, as described below, when tension is applied to the elastic body 5, sliding up of the elastic body attaching portion 31 (in other words, the lower limb mounting portion 3) upward in the vertical direction V can be further regulated.

In the embodiment, the elastic body attaching portion 31 is integrally molded with other parts of the lower limb mounting portion 3. However, as long as the relative movement in the vertical direction V of the elastic body attaching portion 31 with respect to the hard frame 4 is regulated, parts other than the elastic body attaching portion 31 of the lower limb mounting portion 3 may be formed with a material other than the hard material. For example, the lower limb mounting portion 3 may be structured so as to have the elastic body attaching portion 31 formed of the hard material and formed integrally with the hard frame 4, and a flexible belt for lower limb (not shown) to be wound around the lower limb L1, L2, which belt for lower limb is separate from the elastic body attaching portion 31 and is formed of a material similar to that for the waist part belt 22. In this case, tension of the elastic body 5 is applied to the elastic body attaching portion 31 but not to the belt for lower limb. Therefore, sliding up of the belt for lower limb does not occur even when the belt for lower limb is wound to the lower limb L1, L2. Moreover, in this case, sliding up of the belt for lower limb does not occur, so that the belt for lower limb does not need to be firmly wound to the lower limb L1, L2 more than necessary.

Although a material forming the lower limb mounting portion 3 is not particularly limited, the lower limb mounting portion 3 is preferably formed of a hard material having a predetermined rigidity, for example. The material forming the lower limb mounting portion 3 may be configured to be, for example, metal such as aluminum, iron, titanium, and the like, or hard resin such as nylon, vinyl chloride, acryl, ABS, polycarbonate, polypropylene, polyethylene, carbon, fiber-reinforced plastic (FRP), and the like. In the embodiment, the entirety of the lower limb mounting portion 3 is formed of the hard material. However, a part (for example, the elastic body attaching portion 31) of the lower limb mounting portion 3 may be formed of the hard material and other parts of the lower limb mounting portion 3 may be formed of material other than the hard material. Please note that the lower limb mounting portion 3 does not necessarily need to be formed of hard material since the effect of sliding up of the lower limb mounting portion 3 is obtained by the hard frame 4 as described below.

In the embodiment, the lower limb mounting portion 3 is configured to be mounted to a position in the vicinity of a knee N in the lower limb L1, L2. Specifically, the lower limb mounting portion 3 is mounted to the thigh T above the knee N. The lower limb mounting portion 3 is preferably configured to mounted to a region between an intermediate position of the root of the lower limb L1, L2 and the knee N, and the knee N, for example. The lower limb mounting portion 3 may be provided below the knee N.

Moreover, although not particularly shown, the lower limb mounting portion 3 may be configured to adjust the position of the lower limb mounting portion 3 in the vertical direction V. For example, the lower limb mounting portion 3 may be connected to the hard frame 4 so as to adjust the position of the lower limb mounting portion 3 in the vertical direction V with respect to the hard frame 4. In this case, in accordance with the height or the body shape of the user, the lower limb mounting portion 3 can be positioned at a position being optimal in the vertical direction V. The structure of a position adjusting mechanism to adjust the position of the lower limb mounting portion 3 is not particularly limited. For example, the lower limb mounting portion 3 may be configured to relatively move in the vertical direction V with respect to the hard frame 4, and the position of the lower limb mounting portion 3 with respect to the hard frame 4 may be fixed by a predetermined fixing tool after adjusting the position of the lower limb mounting portion 3 with respect to the hard frame 4 to adjust the position of the lower limb mounting portion 3.

As shown in FIGS. 4 to 6, the elastic body 5 is connected to the waist part mounting portion 2 and the lower limb mounting portion 3. The elastic body 5 extends from the waist part mounting portion 2 toward the lower limb mounting portion 3 on the back side of the user. Therefore, as described below, the elastic body 5 is elongated when the user bends forward (see FIG. 8), and an assisting force when the user returns the posture from a forward bending posture to an upright posture is produced by the tension of the elongated elastic body 5.

The type of the elastic body 5 is not particularly limited as long as the elastic body 5 is stretchable in a direction in which the waist part mounting portion 2 and the lower limb mounting portion 3 are connected and the elastic body 5 can produce the assisting force when the user returns the posture from the forward bending posture to the upright posture. In the embodiment, the elastic body 5 is formed of a stretchable fabric. In case that the elastic body 5 is formed of a fabric, manufacturing of the elastic body 5 is easy and there is no discomfort when the elastic body 5 is mounted over a clothing. The elastic body 5 may be formed of a rubber tube, a spring, and the like.

In the embodiment, as shown in FIGS. 4 to 6, the elastic body 5 is formed in a shape of a band, and one band-shaped elastic body 5 extends while being folded back in a V-shape. In more detail, the elastic body 5 extends from the waist part mounting portion 2 to the first lower limb mounting portion 3a, passing along the buttock G and the back of the thigh T. The elastic body 5 is folded back at the elastic body attaching portion 31 of the first lower limb mounting portion 3a and extends to the upper frame 21a of the waist part mounting portion 2, passing along the back of the thigh T, and the buttock G. The elastic body 5 is again folded back at the upper frame 21a and extends to the second lower limb mounting portion 3b, passing along the buttock G and the back of the thigh T. The elastic body 5 is further folded back at the elastic body attaching portion 31 of the second lower limb mounting portion 3b to be connected to the lower frame 21b of the waist part mounting portion 2. The shape of the elastic body 5 is not limited. The elastic body 5 may be formed of an elastic body in a shape of one sheet so as to cover the entirety of the buttocks G and the upper portion of the lower limb L1, L2.

In the embodiment, as shown in FIGS. 4 to 6, the elastic body 5 comprises a length adjusting mechanism 51 by which a length of the elastic body 5 is adjustable to adjust a tension of the elastic body 5. Although the structure of the length adjusting mechanism 51 is not particularly limited, the length adjusting mechanism 51 may be configured to be a buckle through which the elastic body can be passed so as to allow the length thereof to be adjusted, for example. This makes it easy to adjust tension of the elastic body 5 in accordance with the user, or the task content of the user.

Moreover, the elastic body 5 may be configured to be attachable to and detachable from the assisting device for waist part 1. This makes replacement of the elastic body 5 or maintenance thereof, such as cleaning, easy.

The hard frame 4 is a frame formed of a hard material and extending between the waist part mounting portion 2 and the lower limb mounting portion 3. When the user is brought to be in a forward bending posture, for example, the elastic body 5 is elongated and a tension is applied to the elastic body 5. With the rigidity of the hard frame 4, the hard frame 4 supports a force applied to the lower limb mounting portion 3 upward in the vertical direction V caused by the tension of the elastic body 5. In this way, sliding up of the lower limb mounting portion 3 caused by the tension of the elastic body 5 is suppressed, bringing a feeling of wearing of the assisting device for waist part 1 to be good and making it possible to continuously obtain a desired assisting force by the elastic body 5. Details of this point will be described below.

The hard material forming the hard frame 4 is not particularly limited as long as the hard frame 4 has the rigidity that can support the tension of the elastic body 5. For example, the hard material of the hard frame 4 may be configured to be metal such as aluminum, iron, titanium, and the like, or hard resin such as nylon, vinyl chloride, acryl, ABS, polycarbonate, polypropylene, polyethylene, carbon, fiber-reinforced plastic (FRP), and the like.

In the embodiment, as shown in FIGS. 1 and 2, the hard frame 4 comprises a first hard frame 4a extending from the waist part mounting portion 2 to the first lower limb mounting portion 3a, passing along the latera side of the first lower limb L1 and a second hard frame 4b extending from the waist part mounting portion 2 to the second lower limb mounting portion 3b, passing along the lateral side of the second lower limb L2. "Lateral side" refers to a side of the left-right direction of the human body, which is farther from the midline of the user. "Medial side" refers to a side of the left-right direction of the human body, which side is nearer to the midline of the user.

In the embodiment, as shown in FIGS. 3 and 5, the hard frame 4 is formed in a shape of a narrow width plate extending in the vertical direction V along the lateral side of the lower limb L1, L2. The shape of the hard frame 4 is not particularly limited as long as the hard frame 4 comprises a portion extending between the waist part mounting portion 2 and the lower limb mounting portion 3 and can support the tension of the elastic body 5. For example, the hard frame 4 may be formed in a shape of a narrow width rod extending in the vertical direction V along the lateral side of the lower limb L1, L2. In case that the hard frame 4 is formed in the shape of the narrow width plate or in the shape of the narrow width rod extending in the vertical direction V along the lateral side of the lower limb L1, L2, the entire assisting device for waist part 1 may be reduced in weight and user's walking is not disturbed. Other than the above-described configuration, the hard frame 4 may be configured to partially cover the front and/or the back of the lower limb L1, L2, for example.

The hard frame 4 is connected to the waist part mounting portion 2. In the embodiment, the hard frame 4 is connected to the base portion 21 formed of the hard material. By connecting the base portion 21 and the hard frame 4, both of which are formed of the hard material, the tension by the elastic body 5 can be easily supported.

In the embodiment, as shown in FIG. 4 and 6, the waist part mounting portion 2 and the hard frame 4 are connected by a false joint portion 6. The false joint portion 6 allows joint movement of the hard frame 4 with respect to the waist part mounting portion 2. This increases the degree of freedom of movement of the lower limb mounting portion 3 and the hard frame 4 that move to follow the movement of the lower limb L1, L2 when the user with the assisting device for waist part 1 being mounted thereto moves the lower limb L1, L2.

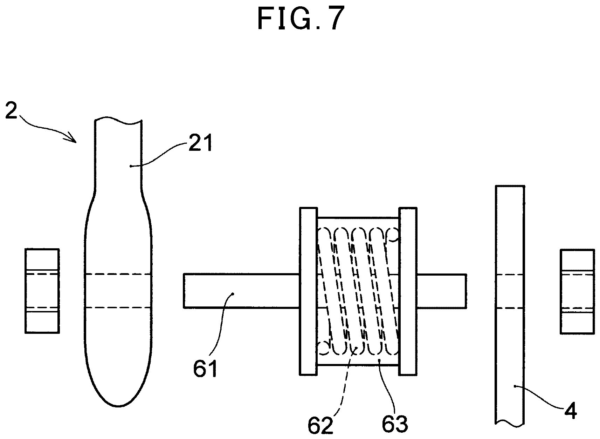

In the embodiment, as shown in FIGS. 4 and 6, a pair of false joint portions 6 are provided to respective positions corresponding to hip joints of lower limbs L1, L2. As shown in FIG. 7, the false joint portions 6 each comprises a shaft portion 61 extending in a direction from the lateral side of the lower limb L1, L2 toward the medial side of the lower limb L1, L2; a coil spring 62 arranged coaxially with the shaft portion 61; and a rubber body 63 covering the coil spring 62. In case of the above-mentioned structure, in the same manner as the hip joint of the human body, free movement of the lower limb L1, L2, such as external rotation, internal rotation, abduction, adduction, and the like, is made possible. Moreover, restoring to the initial state becomes easy by the elastic force of the coil spring 62 and the rubber body 63 after the hard frame 4 moves from the initial state. The structure of the false joint portion 6 is not particularly limited, so that, for example, the false joint portion may realize a movement in the same manner as the hip joint using a ball joint, for example.

Next, exemplifying the assisting device for waist part 1 in the embodiment, operational advantages of the assisting device for waist part 1 will be described in more detail. The example below is merely one example, so that the invention is not limited by the example below.

First, in case that the assisting device for waist part 1 is mounted, the user winds the waist part belt 22 around the waist part W with the base portion 21 being pressed against the waist part W over a clothing. Next, the tightening belt 23 is tightened to fix the waist part mounting portion 2 to the waist part W. Moreover, for example, the front of the lower limb mounting portion 3 is opened to be attached to the thigh T of the lower limb L1, L2 and the front of the lower limb mounting portion 3 is closed and fixed, allowing mounting of the assisting device for waist part 1 to be completed (see FIGS. 1 to 3).

When the user walks after the mounting of the assisting device for waist part 1 is completed, as shown in FIGS. 1 and 3, the hard frame 4 is positioned at lateral side of the lower limb L1, L2 of the user, so that it does not obstruct the walking. Moreover, when the user carries out various movements such as walking, spreading of the lower limbs L1, L2, and the like, the hard frame 4 and the lower limb mounting portion 3 follow the movement of the lower limb L1, L2 since the hard frame 4 and the waist part mounting portion 2 are connected by the false joint portion 6. Thus, a smooth movement of the user is not disturbed by the assisting device for waist part 1.

In case that the user with the assisting device for waist part 1 lifts a heavy object , as shown in FIG. 8, the user is brought to be in a forward bending posture. When the user is brought to be in the forward bending posture, the waist part mounting portion 2 rotates around the shaft portion 61 of the false joint portion 6 in conjunction with a forward inclining movement of the waist part W of the user. As shown in FIG. 8, a part of the elastic body 5 connected to the waist part mounting portion 2 and a part of the elastic body 5 connected to the elastic body attaching portion 31 move forward with respect to those in the upright posture shown in FIG. 3. At the same time, the central portion of the elastic body 5 in the vertical direction V deflects and deforms backward in conjunction with sticking out backward of the buttocks G of the user when the user is brought to be in the forward bending posture. In this way, the elastic body 5 elongates and is tensioned. At this time, a force to push back forward the buttocks G shown in FIG. 8 with an arrow A1 and a force to pull back the waist part W shown with an arrow A2 act on the user by the elastic body 5. This makes it possible to obtain an assisting force by the elastic body 5 when the user transitions from the forward bending posture to the upright posture.

As mentioned above, the elastic body 5 elongates when the user is brought to be in the forward bending posture and a force shown in FIG. 8 with an arrow A3 is applied to the lower limb mounting portion 3 upward in the vertical direction V. The above-mentioned force shown with the arrow A3 is supported by the hard frame 4, and the movement of the lower limb mounting portion 3 upward in the vertical direction V is regulated and the lower limb mounting portion 3 is held at a predetermined position. Therefore, it is suppressed that the lower limb mounting portion 3 moves upward in the vertical direction V and that an elongation amount of the elastic body 5 decreases (an assisting force due to tension decreases) by the lower limb mounting portion 3 moving upward. Thus, the elongation amount of the elastic body 5 is not changed when the user is brought to be in the forward bending posture and a desired assisting force by the elastic body 5 can be continuously obtained. Moreover, by providing the hard frame 4, sliding up of the lower limb mounting portion 3 along with the clothing over the clothing is suppressed. Therefore, it is suppressed that the user feels uncomfortable due to sliding up of the lower limb mounting portion 3 at the time of mounting of the assisting device for waist part 1. Moreover, as sliding up of the lower limb mounting portion 3 is suppressed by the hard frame 4, there is no need to firmly tighten the lower limb mounting portion 3 with respect to the thigh T. Therefore, it is possible to loosely wind the lower limb mounting portion 3 around the thigh T or to mount the lower limb mounting portion 3 with a gap with respect to the thigh T. Therefore, a feeling of wearing can be brought to be good.

Moreover, by providing the hard frame 4, a part of the force applied to the hip joint of the human body in the vertical direction V is supported by the hard frame 4 when the user holds a heavy object in the forward bending and upright postures. Therefore, a force applied to the hip joint of the human body is alleviated, and the user damaging the hip joint is suppressed.

Furthermore, in the embodiment, the assisting device for waist part 1 does not have any constituting component on the side upper to the waist part mounting portion 2. In other words, the assisting device for waist part 1 does not have a portion applying load on the side upper to the waist part W of the user (for example, an elastic body, a portion to be put on the upper back, a portion to be placed on the shoulder, and the like). Thus, the human assisting device 1 applying no load on the upper half of the human body, such as the shoulder and the upper back, makes it possible to decrease the burden on the user.

One embodiment of the invention has been described above. The invention is not particularly limited to the above-described embodiment. Besides, the above-described embodiment is to mainly describe the invention having the configuration below.
(1) An assisting device for waist part comprising: a waist part mounting portion to be mounted to the waist part of a user; a lower limb mounting portion to be mounted to a part of a lower limb of the user; a hard frame extending between the waist part mounting portion and the lower limb mounting portion; and an elastic body extending from the waist part mounting portion toward the lower limb mounting portion on a back side of the user.
   According to such a configuration, it is possible to bring a feeling of wearing to be good and to continuously obtain a desired assisting force.
(2) The assisting device for waist part according to (1), wherein the lower limb mounting portion comprises an elastic body attaching portion to which the elastic body is attached, the elastic body attaching portion is formed with a hard material, and the elastic body attaching portion is connected to the hard frame in a state in which a relative movement of the elastic body attaching portion in a vertical direction with respect to the hard frame is regulated.
   According to such a configuration, it is possible to bring a feeling of wearing the lower limb mounting portion to be better.
(3) The assisting device for waist part according to (1) or (2), wherein the waist part mounting portion comprises a base portion formed of a hard material, and the hard frame is connected to the base portion.
   According to such a configuration, the effect of the hard frame can be easily obtained by connecting the base portion of the waist part mounting portion and the hard frame.
(4) The assisting device for waist part according to any one of (1) to (3), wherein the waist part mounting portion and the hard frame are connected by a false joint portion.
   According to such a configuration, the degree of freedom of movement of the hard frame with respect to the waist part mounting portion increases.
(5) The assisting device for waist part according to (4), wherein a pair of false joint portions are provided to respective positions corresponding to hip joints of lower limbs; and the pair of false joint portions each comprises a shaft portion extending in a direction from a lateral side of the lower limb toward a medial side of the lower limb; a coil spring arranged coaxially with the shaft portion; and a rubber body covering the coil spring.
   According to such a configuration, by the false joint, free movement in the same manner as the hip joint of the human body is made possible, and, moreover, restoring to the initial state becomes easy after the hard frame moves from the initial state.
(6) The assisting device for waist part according to (1) to (5), wherein the lower limb mounting portion comprises a first lower limb mounting portion to be mounted to a first lower limb and a second lower limb mounting portion to be mounted to a second lower limb; the hard frame comprises a first hard frame extending from the waist part mounting portion to the first lower limb mounting portion, passing along a lateral side of the first lower limb; and a second hard frame extending from the waist part mounting portion to the second lower limb mounting portion, passing along a lateral side of the second lower limb.
   According to such a configuration, the hard frame passes along the lateral side of the lower limb of the human body and the forward bending movement is not disturbed, making it possible for a user to perform a smooth operation.
(7) The assisting device for waist part according to any one of (1) to (6), the elastic body is attachable to and detachable from the assisting device for waist part.
   According to such a configuration, replacement, maintenance, and the like of the elastic body becomes easy.
(8) The assisting device for waist part according to any one of (1) to (7), wherein the elastic body comprises a length adjusting mechanism by which a length of the elastic body is adjustable to adjust a tension of the elastic body
   According to such a configuration, it is easy to adjust tension of the elastic body in accordance with the user or the task.
(9) The assisting device for waist part according to (1) to (8), wherein the elastic body is formed of a fabric.
   According to such a configuration, manufacturing of the elastic body is easy and there is no discomfort when the elastic body 5 is mounted over a clothing.

### EXPLANATION OF NUMERALS

1 ASSISTING DEVICE FOR WAIST PART
2 WAIST PART MOUNTING PORTION
21 BASE PORTION
21a UPPER FRAME
21b LOWER FRAME
21c REINFORCING FRAME
22 WAIST PART BELT
23 TIGHTENING BELT
3 LOWER LIMB MOUNTING PORTION
3a FIRST LOWER LIMB MOUNTING PORTION
3b SECOND LOWER LIMB MOUNTING PORTION
31 ELASTIC BODY ATTACHING PORTION
4 HARD FRAME
4a FIRST HARD FRAME
4b SECOND HARD FRAME
5 ELASTIC BODY
51 LENGTH ADJUSTING MECHANISM
6 FALSE JOINT PORTION
61 SHAFT PORTION
62 COIL SPRING
63 RUBBER BODY
G BUTTOCKS
L1, L2 LOWER LIMB
N KNEE
T THIGH
V VERTICAL DIRECTION
W WAIST PART
W1, W2 BOTH SIDE PORTIONS OF WAIST PART

## Claims

1. An assisting device for waist part comprising:
a waist part mounting portion to be mounted to the waist part of a user;
a lower limb mounting portion to be mounted to a part of a lower limb of the user;
a hard frame extending between the waist part mounting portion and the lower limb mounting portion; and
an elastic body extending from the waist part mounting portion toward the lower limb mounting portion on a back side of the user.

2. The assisting device for waist part according to claim 1, wherein the lower limb mounting portion comprises an elastic body attaching portion to which the elastic body is attached, the elastic body attaching portion is formed with a hard material, and the elastic body attaching portion is connected to the hard frame in a state in which a relative movement of the elastic body attaching portion in a vertical direction with respect to the hard frame is regulated.

3. The assisting device for waist part according to claim 1 or 2, wherein the waist part mounting portion comprises a base portion formed of a hard material, and the hard frame is connected to the base portion.

4. The assisting device for waist part according to any one of claims 1 to 3, wherein the waist part mounting portion and the hard frame are connected by a false joint portion.

5. The assisting device for waist part according to claim 4, wherein a pair of false joint portions are provided to respective positions corresponding to hip joints of lower limbs; and
the pair of false joint portions each comprises
a shaft portion extending in a direction from a lateral side of the lower limb toward a medial side of the lower limb;
a coil spring arranged coaxially with the shaft portion; and
a rubber body covering the coil spring.

6. The assisting device for waist part according to any one of claims 1 to 5, wherein
the lower limb mounting portion comprises a first lower limb mounting portion to be mounted to a first lower limb and a second lower limb mounting portion to be mounted to a second lower limb;
the hard frame comprises a first hard frame extending from the waist part mounting portion to the first lower limb mounting portion, passing along a lateral side of the first lower limb; and a second hard frame extending from the waist part mounting portion to the second lower limb mounting portion, passing along a lateral side of the second lower limb.

7. The assisting device for waist part according to any one of claims 1 to 6, wherein the elastic body is attachable to and detachable from the assisting device for waist part.

8. The assisting device for waist part according to any one of claims 1 to 7, wherein the elastic body comprises a length adjusting mechanism by which a length of the elastic body is adjustable to adjust a tension of the elastic body.

9. The assisting device for waist part according to any one of claims 1 to 8, wherein the elastic body is formed of a fabric.
